(19) Europäisches Patentamt
European Patent Office
Office européen des brevets

(11) Publication number: 0 515 133 A2

# (12) EUROPEAN PATENT APPLICATION

(21) Application number: **92304501.7**

(22) Date of filing: **19.05.92**

(51) Int. Cl.5: **C07D 311/82**, C07D 311/84, G01M 3/20, G03F 7/031, G01T 1/06

(30) Priority: **20.05.91 US 702886**
**09.09.91 US 756611**
**07.10.91 US 772103**

(43) Date of publication of application:
**25.11.92 Bulletin 92/48**

(84) Designated Contracting States:
**BE CH DE FR GB IT LI NL SE**

(71) Applicant: **SPECTRA GROUP LIMITED INC**
**1722 Indian Wood Circle, Suite H**
**Maumee, Ohio 43537(US)**

(72) Inventor: **Neckers, Douglas C.**
**108 Secor Woods Lane**
**Perrysburg, Ohio 43551(US)**
Inventor: **Shi, Jianmin**
**214 Napoleon Road, Apt.14**
**Bowling Green, Ohio 43402(US)**

(74) Representative: **Deans, Michael John Percy**
**Lloyd Wise, Tregear & CO. Norman House**
**105-109 Strand**
**London WC2R OAE(GB)**

## (54) Fluorone and pyronin Y derivatives.

(57) A novel class of compounds is described, certain members of which are useful as fluorescers or as photoinitiators. The compounds have the general structures (I) (II) (III) or (IV):

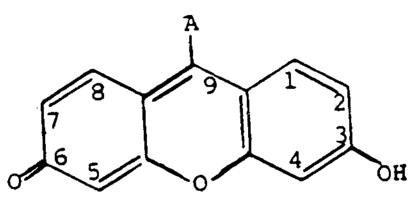

(I)

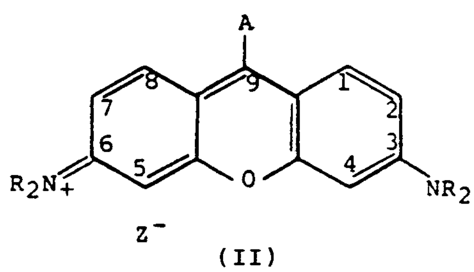

(II)

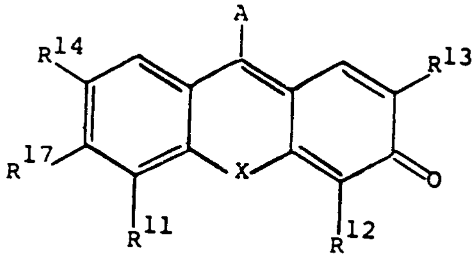

(III)

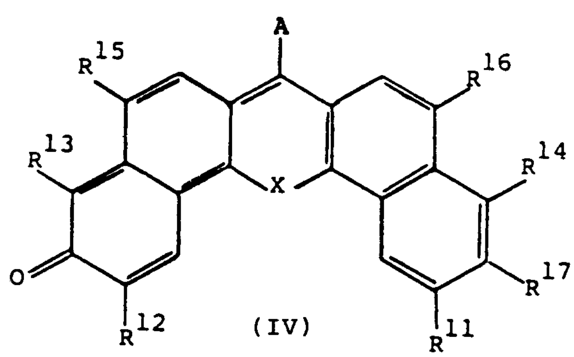

(IV)

where A is selected from hydrogen,



Rank Xerox (UK) Business Services

alkyl, aryl, alkenyl, alkynyl, dichlorotriazinylamino, and electron withdrawing groups (EWG) which are stronger electron withdrawing groups than a phenyl group or a halogen-substituted or a carboxyl-substituted phenyl group; X is oxygen, sulfur, selenium, tellurium or >C=0 and preferably oxygen; Z is a counter ion; R is hydrogen, alkyl, aryl or aralkyl; $R^{11}$-$R^{16}$ are the same or different and represent a hydrogen atom or a halogen atom; and $R^{17}$ is a hydrogen atom or a group of the formula -OR'.

The compounds of formula (I) and (II) may be substituted or unsubstituted at the 2, 4, 5 and 7 positions. When the 2, 4, 5 and 7 positions are unsubstituted, A cannot be hydrogen or methyl.

The present invention relates to a novel class of compounds, examples of which are useful as fluorescers or as photoinitiators.

Examples of our compounds described in more detail below have the general structures (I) (II) (III) or (IV):

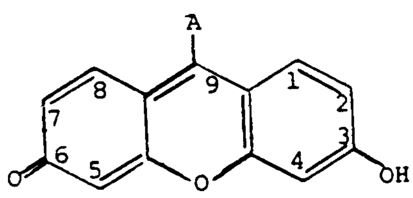

(I)

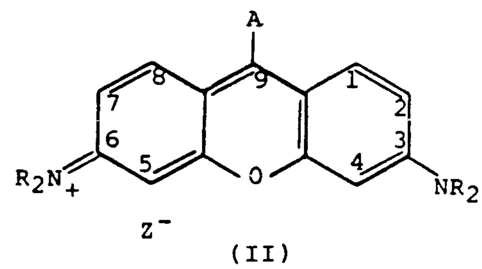

(II)

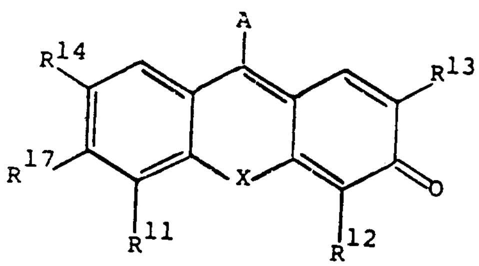

(III)

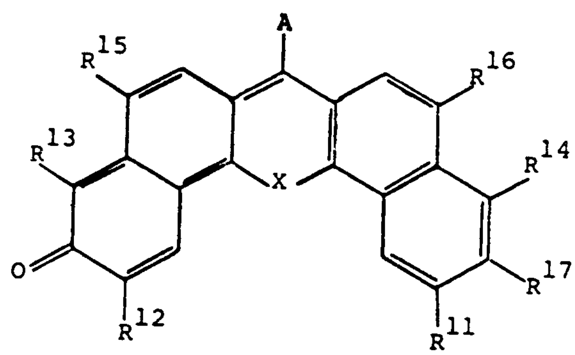

(IV)

where A is selected from hydrogen, alkyl, aryl, alkenyl, alkynyl, dichlorotriazinylamino, and electron withdrawing groups (EWG) which are stronger electron withdrawing groups than a phenyl group or a halogen-substituted or a carboxyl-substituted phenyl group; X is oxygen, sulfur, selenium, tellurium or >C=0 and preferably oxygen; Z is a counter ion; R is hydrogen, alkyl, aryl or aralkyl; $R^{11}$-$R^{16}$ are the same or different and represent a hydrogen atom or a halogen atom; and $R^{17}$ is a hydrogen atom or a group of the formula -OR'.

The compounds of formula (I) and (II) may be substituted or unsubstituted at the 2, 4, 5 and 7 positions. When the 2, 4, 5 and 7 positions are unsubstituted, A cannot be hydrogen or methyl. In one embodiment the compound is substituted at A or the 2, 4, 5 or 7 position with a functional group useful in linking the compound to an antibody or a ligand analog for use in immunoassays. In another embodiment the compounds are substituted with oleophilic moieties which make the compounds soluble in oil and useful as fluorescent dyes for oil leak detection. In another embodiment, the 2, 4, 5 and 7 positions may be substituted by bromine or iodine atoms as in formulas (III) and (IV) to provide compounds useful as photoinitiators.

Depending upon structure and substitution, specific examples of our compounds absorb visible light or infrared radiation and fluoresce or generate free radicals. As contrasted with conventional fluorescein dyes, the addition of the A substituent and, more particularly, the stronger EWG at the 9 position extends the wavelength of maximum absorption approximately 100 nm to longer wavelengths.

In accordance with a first aspect of the present invention, we provide a compound of the formula:

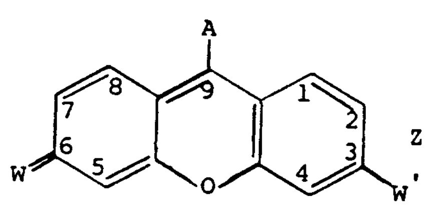

where A is hydrogen, alkyl, alkenyl, alkynyl, dichlorotriazinylamino, or an electron withdrawing group (EWG) which is more strongly electron withdrawing than a phenyl or a substituted phenyl group, W and W' are respectively selected from O and $O^-$, or $NR_2^+$ and $NR_2$, R is hydrogen, lower alkyl, aralkyl, or aryl, and Z is a hydrogen atom or a counter ion, and the compound may be substituted or unsubstituted at the 2, 4, 5 and 7 positions, provided that when A is hydrogen or methyl, the compound is substituted at at least one of the 2, 4, 5 and 7 positions.

A preferred group of our compounds are those represented by the formula (V):

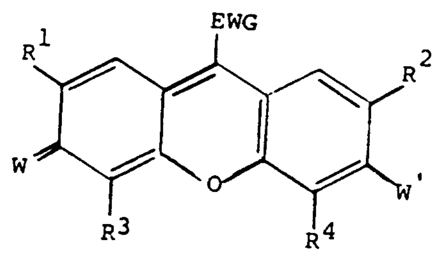

(V)

where EWG is an electron withdrawing group selected from COOR, $COR^5$, C(O)OCOR, $CONR_2$, CN, $NO_2$, NCS, NCO, $SO_2R^5$ $SO_3R$, $SO_2NR_2$, $CX_3$; R is hydrogen, alkyl, aryl, or aralkyl; X can be the same or different and is a halogen atom; W and W' are O and O- or $NR_2$ + and $NR_2$ where R is defined as above; $R^1$-$R^4$ may be the same or different and are selected from hydrogen, halogen, alkyl, alkenyl, aryl, aralkyl, alkaryl, $SO_3R$, $SO_2R^5$, $SO_2NR_2$, $NO_2$, NCO, NCS, $(CH_2)_nNCO$, $(CH_2)_nNCS$, CN, $(CH_2)_nCN$, $(CH_2)_nCOOR^5$, $(CH_2)_nCONR_2$, $(CH_2)_nX$, $(CH_2)_nNR_2$; where n is 1 to 6, and $R^3$ and $R^4$ may combine to form a 10 to 12 membered ring; and $R^5$ is hydrogen, alkyl, aryl, aralkyl, N-imido such as N-succinimido and N-maleimido, or $NR_2$. Still more specifically, examples of our compounds may be represented by the formulas VI and VII.

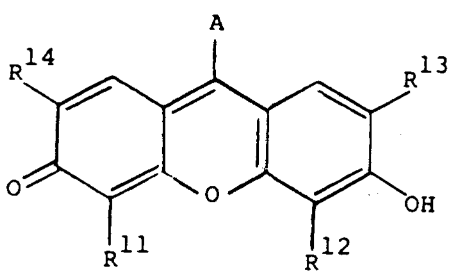

(VI)

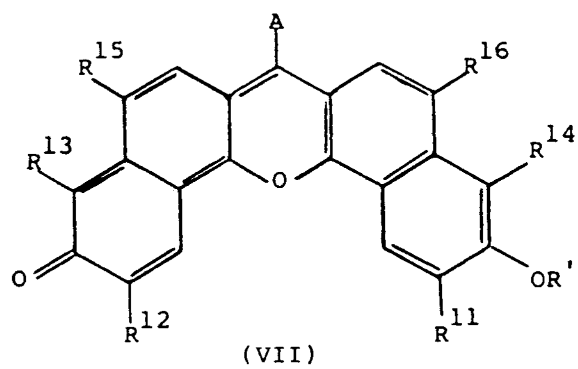

(VII)

where R' and $R^{11}$-$R^{16}$ are defined as above.

When $R^5$ is N-imido or $NR_2$ the compounds are esters of N-hydroxyimides such as N-hydroxysuccinimides or N-hydroxyamines. Typically, $R^1$ and $R^2$ are the same and $R^3$ and $R^4$ are the same.

For use in immunoassays, at least one of $R^1$-$R^4$ preferably includes a functional group such as an amino group, a carboxyl group, a sulfinyl group, a sulfonyl group, an isocyanato group, an isothiocyanato group, a halogen atom, etc. which enables the dye to be coupled with a ligand analog, an antibody or similar substrate.

For use in oil leak detection, $R^1$-$R^4$ may be selected to give the dye a desired degree of oil solubility. The addition of alkyl groups at the 2 and 7 positions extends absorption in the red at least an additional 20 nm, increases the fluorescence quantum yield, and decreases the bleaching rate.

For use as a photoinitiator, at least one of the $R^{11}$-$R^{16}$ must be halogen. Examples of halogen atoms represented by $R^{11}$-$R^{16}$ are chlorine, bromine and iodine. In one embodiment $R^{11}$-$R^{16}$ in formula (VI) and $R^{11}$-$R^{14}$ in formula (VII) are the same and represent bromine or iodine. In another embodiment $R^{12}$ and $R^{13}$ in formula (VII) and $R^{11}$ and $R^{16}$ in formula (VI) are halogen and the balance of $R^{11}$-$R^{16}$ are hydrogen.

Examples of specific photoinitiators in accordance with the present invention include 5,7-diiodo-3-methoxy-6-fluorone, 2,4,5,7-tetraiodo-3-hydroxy-6-fluorone, 2,4,5,7-tetrabromo-3-hydroxy-6-fluorone, 9-cyano-3-hydroxy-2,4,5,7-tetraiodo-6-fluorone, and 9-cyano-3-hydroxy-2,4,5,7-tetrabromo-3-hydroxy-6-fluorone.

To exhibit fluorescence, the compounds must be ionized. In another embodiment, $R^2$ or $R^4$ includes a moiety which produces intramolecular ionization of the compound at W/W'.

Thus, in a second and alternative aspect of the present invention, there is provided

a compound of the formula (III) or (IV)

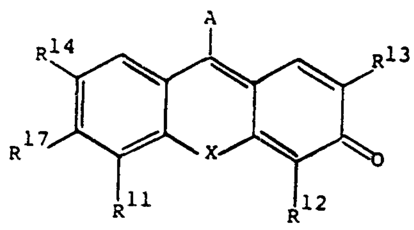

(III)

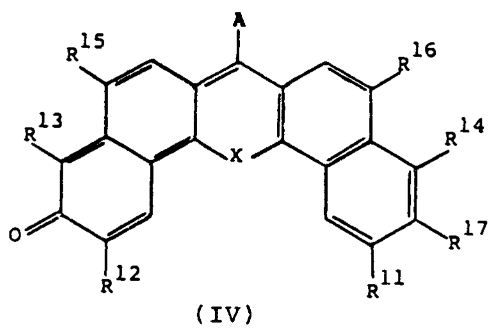

(IV)

where X is oxygen, sulfur, selenium, tellurium, or C=0, A is hydrogen, alkenyl, aryl, or an electron withdrawing group selected from quaternary amino,
nitro, nitroso, cyano, $-SO_3R'$, $-SO_2R'$, halogen,
trihalomethyl, -CHO, carbamoyl, sulfamoyl, sulfinyl and -COOR'

where R' is hydrogen, alkyl, aryl, or aralkyl; and $R^{11}$-$R^{16}$ are the same or different and represent a hydrogen or a halogen atom; and $R^{17}$ is a hydrogen atom or an -OR' group.

The invention provides, according to a third alternative aspect thereof,

a composition for detecting oil leakage comprising an oil vehicle and a fluorescent dye, characterised in that said dye is a compound of the formula:

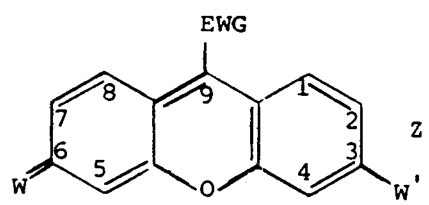

where EWG is an electron withdrawing group which is more strongly electron withdrawing than a phenyl or a halogen or carboxyl substituted phenyl group, W and W' are O and $O^-$ or $NR_2^+$ and
$NR_2$, R is hydrogen, alkyl, aralkyl, or aryl, Z is a hydrogen atom or a counter ion, and the compound is substituted with an oleophilic moiety at at least one of the 2, 4, 5 and 7 positions.

In accordance with the more preferred embodiments of the invention, the compounds are represented by the formula (VIII) and (IX):

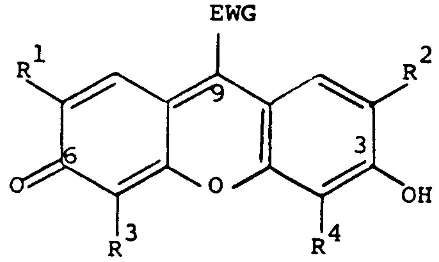

(VIII)

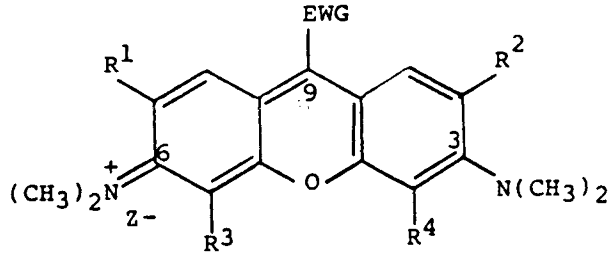

(IX)

The invention also provides tracers useful in immunoassays of the formula (X):

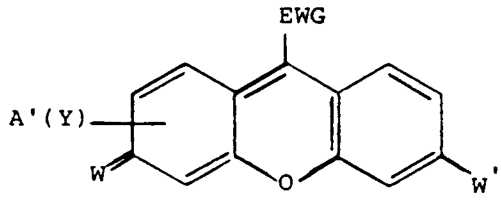

(X)

where Y is a linking group and A' is a ligand-analog as defined in U.S. Patent 4,585,862 or an antibody, and EWG, W and W' are defined as above. The disclosure of U.S. Patent 4,585,862 is to be regarded as incorporated herein by reference.

In accordance, therefore, with a yet further alternative aspect of this invention, we provide a compound of the formula (X):

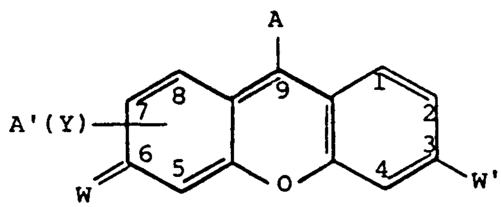

(X)

where A is selected from hydrogen, alkenyl, alkynyl, dichlorotriazinylamino, COOR, $COR^5$, $CONR_2$, C(O)OCOR, CN, $NO_2$, NCS, NCO, $SO_2R^5$, $SO_3R$, $SO_2NR_2$, $CX_3$ where R is hydrogen, alkyl, aryl, or aralkyl and X may be the same or different and is a halogen atom; W and W' are selected from either O and O-, or $NR_2$+ and $NR_2$; $R^5$ is hydrogen, alkyl, aryl, aralkyl, N-imido or $NR_2$; and A' represents a ligand-analog or an antibody and Y is a linking group and the A'(Y) moiety may be at one of the 2, 4, 5, 7 or 9 positions and the balance of those positions may be substituted or unsubstituted.

In one embodiment, the linking group Y is the divalent analog of one of the aforesaid functional $R^1$-$R^4$ groups. Particularly useful linking groups include an oxalyl group, a sulfonyl group, a hydroxyimido group, or a carboamidosulfonyl group. In another embodiment the antibody or ligand-analog may be linked to the tricyclic nucleus via the moiety X' in formula (VII) below. In still another embodiment linkage may be via the A moiety at the 9-position. One compound which may be useful in immunoassays is substituted by hydrogen at the 9-position and cyano, isocyanato or isothiocyanato at one of the 2, 4, 5 and 7 positions.

Representative examples of alkyl groups represented by R and $R^1$-$R^5$ are straight chain, branched chain and cyclic alkyl groups having 1 to 10 carbon atoms.

Representative examples of aryl groups represented by R and $R^1$-$R^5$ include phenyl groups which may be unsubstituted or substituted by alkyl, $(CH_2)_p$ COOR, $(CH_2)_pX$, $(CH_2)_pNR_2$, where R and X are defined as above and p is 0 to 6.

Representative examples of aralkyl groups represented by $R^1$-$R^5$ include aralkyl groups containing 7 to 20 carbon atoms such as benzyl, phenethyl, etc.

Representative examples of alkaryl groups include alkaryl groups containing 7 to 20 carbon atoms such as phenyl substituted at the ortho or para position by a straight chain or branched chain alkyl group having 1 to 6 carbon atoms.

Representative examples of alkenyl groups represented by EWG or $R^1$-$R^5$ include alkenyl groups having 2 to 10 carbon atoms such as vinyl, allyl, 1-propenyl, 1-butenyl and 1,3-butadienyl.

Representative examples of alkynyl groups represented by EWG include alkynyl groups having up to 10 carbon atoms such as 1-propynyl, 1-butynyl, etc.

Representative examples of the halogen atoms represented by X or $R^1$-$R^5$ and $R^{11}$-$R^{16}$ include fluorine, chlorine, bromine and iodine and preferably fluorine or chlorine in the case of fluorescers and bromine or

iodine in the case of photoinitiators.

When $R^3$ and $R^4$ combine to form a ring, one class of such compounds can be represented by the formula XI.

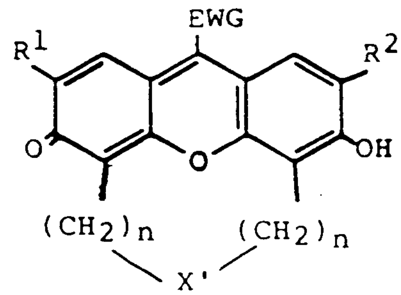

(XI)

where EWG, $R^1$ and $R^2$ are defined as above, n is 2 or 3, and X' is NH, $NR^6$, O, $PR^6$, $CHR^6$ where $R^6$ has the same definition as $R^1$-$R^4$ but can be the same as or different from $R^1$-$R^4$ and may additionally represent dichlorotriazinyl. For intramolecular ionization at least one of $R^1$-$R^4$ will have a basic functional group when $W/W^1$ is $O/O^-$ and have an acid functional group when $W/W^1$ is $NR_2+/NR_2$. Examples of such moieties are acetyl and amino groups.

Representative examples of the counter ion represented by Z include chloride, bromide, iodide, perchlorate when Z is an anion and monovalent ions such as $K^+$, $Na^+$, ammonium, phosphonium, etc. when Z is a cation. When W/W' is $O/O^-$, Z may be hydrogen.

Examples of our compounds have been found to absorb at wavelengths greater than 550 nm and preferably greater than 580 nm. In most compounds, these longer wavelengths of maximum absorption are believed to be attributed to the presence of a strong EWG like cyano at the 9-position. This effect is illustrated in the following table for compounds of the formula V wherein the substituents are as defined in the following Table:

| | W/W' | A/EWG | $R^1/R^2$ | $R^3/R^4$ | λmax in EtOH | ε |
|---|---|---|---|---|---|---|
| | 1 | 2 | 3 | 4 | 5 | 6 |
| 1 | $O/O^-$ | H | H | H | 504 | 24700 |
| 2 | $O/O^-$ | CN | H | H | 594, 548 | 50300,24700 |
| 3 | $O/O^-$ | H | Br | Br | 530 | 39300 |
| 4 | $O/O^-$ | CN | Br | Br | 626, 576 | 51400, 24500 |
| 5 | $O/O^-$ | H | I | I | 536 | 91200 |
| 6 | $O/O^-$ | CN | I | I | 638, 586 | 80000, 35000 |
| 7 | $O/O^-$ | H | H | I | 520 | 86000 |
| 8 | $O/O^-$ | CN | H | I | 618, 570 | 30500, 16600 |
| 9 | $O/O^-$ | H | t-Bu | H | 518 | 101000 |
| 10 | $O/O^-$ | CN | t-Bu | H | 614,564 | 47400,23200 |
| 11 | $O/O^-$ | H | t-Bu | I | 532 | 90800 |
| 12 | $O/O^-$ | CN | t-Bu | I | 636,582 | 68300, 33600 |

In more preferred embodiments, the compounds absorb strongly in the range of 650-700 nm.

Compounds of the formula (II) can be obtained from 2,2',4,4'-tetrahydroxybenzophenone using the following Reaction Scheme 1:

Reaction Scheme 1

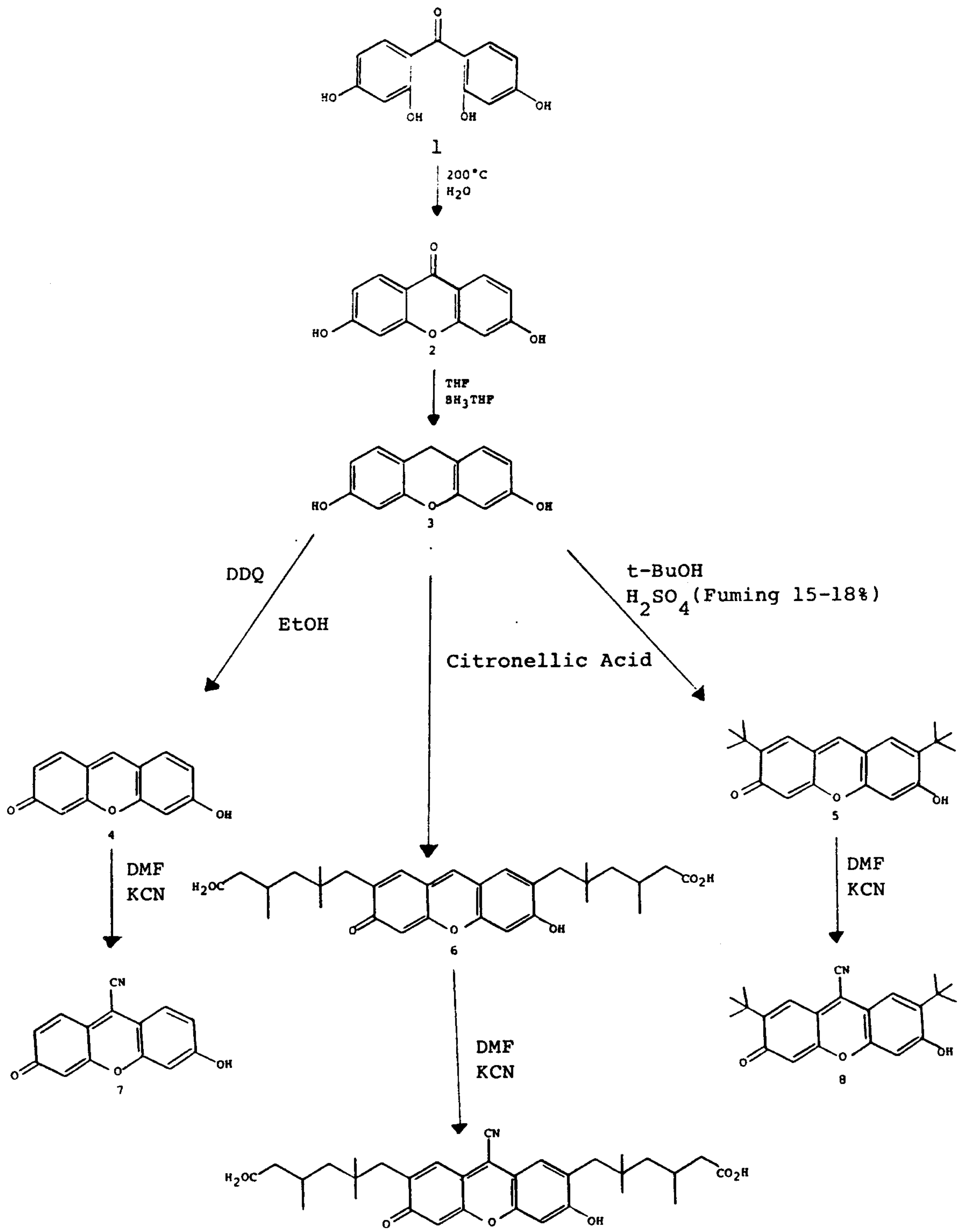


Tetraiodo and tetrabromo hydroxfluorones can be obtained from 3,6-dihydroxyxanthane by the following Reaction Scheme 2:

Reaction Scheme 2

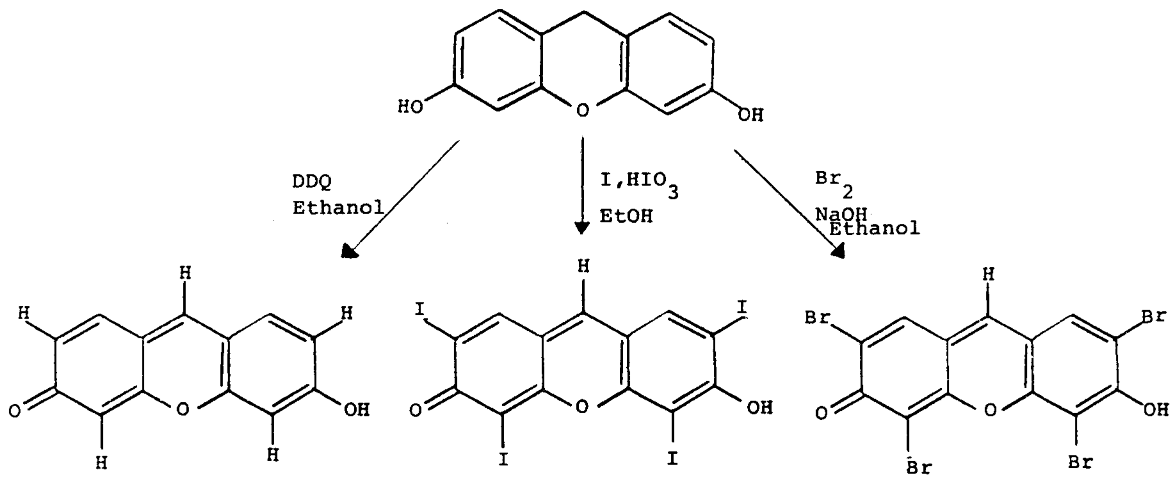


Synthesis Example 1

3,6-Dihydroxyxanthone

2,2',4,4'-tetrahydroxybenzophenone (2.5 g, 10.15 mmol.) was heated in 20 ml of water at 195° C-200° C for 4 hours (Aldrich pressure tube). After cooling, the crude product was filtered off and mixed with 25 ml of water. The resulting suspension was refluxed for 125 min. and filtered at about 60° C. Pure product 3,6-dihydroxyxanthone 2.15 g was obtained in 90.4% yield. It did not melt below 330° C. HNMR (DMSO): 10.780(s,2H), 7.983(d,2H,J = 8.6 Hz), 6,822(m,4H). MS: 228, 200, 171, 115, 100, 69, 63.

Synthesis Example 2

3,6-Dihydroxyxanthane

To a suspension of 3,6-dihydroxyxanthone (46 g, 201 mmol.) in 1.6 L of THF was added over 1 hr. 800 ml of borane-tetrahydrofuran complex (1.0 M in THF, caution very vigorous reaction at beginning). This mixture was stirred overnight under argon at room temperature to give a clear yellow solution. The excess diborane was decomposed by careful addition of water followed by 1N HCl to give a clear yellow solution. The tetrahydrofuran was removed by rotary evaporation and the resulting solid filtered, washed with water and redissolved in 10% NaOH. This solution was filtered, cooled and reprecipitated with slow addition of HCl to give a yellow solid which was filtered, washed with water and dried under vacuum to give 40.14 g (93%, mp 207-208) of 3,6-dihydroxyxanthane. $H^1$NMR (DMSO):9.41 (s, 2H); 6.97 (d, 2H, J = 8.4 Hz), 6.4 (dd, 2H, J = 8.4,2.4 Hz), 6.39 (d, 2H, J = 2.4Hz), 3.765 (s, 2H).

Synthesis Example 3

3-Hydroxy-6-fluorone

To a solution of 3,6-dihydroxyxanthane (2.14 g 10 mmol) obtained as in example 2 in 60 ml of ethanol at 25° C was added DDQ (2.78 g, 12 mmol). The reaction mixture was stirred for 8 hours at room temperature. The yellow precipitate was filtered off and washed with ethanol. 3-Hydroxy-6-fluorone (1.9 g) was obtained in 90% yield. NMR (DMSO):8.203 (s,1H), 7.589 (d,2H, J = 8.6Hz), 6.659 (d,2H, J = 9.2 Hz), 6.478 (s, 2H). λmax = 500 nm (MeOH).

Synthesis Example 4

2,7-Di-t-butyl-3-hydroxy-6-fluorone

8.0 ml of 15-18% fuming sulfuric acid was added dropwise to 3,6-dihydroxyxanthane (10.7 g, 50 mmol.) in 50.0 ml of 2-methyl-2-propanol. The mixture was stirred for 30 minutes and then heated to reflux for one hour. Thereafter 15.0 ml of 2-methyl-2-propanol was added and the solution was refluxed for another 2.5 hours. Upon cooling, the precipitate which formed was filtered and washed with water. 2,7-di-t-butyl-3-hydroxy-6-fluorone (15.8 g) was obtained in 97.5% yield. HNMR(DMSO): 9.11(s,1H), 7.89(s,2H), 7.05(s, 2H), 1.43(s, 18H).

Synthesis Example 5

2,7-Di-t-butyl-4,5-diiodo-3-hydroxy-6-fluorone

Iodic acid (352 mg, 2.0 mmol) dissolved in a minimum amount of water was added dropwise to a solution of 1.62 g (5.0 mmol.) of 2,7-di-t-butyl-3-hydroxy-6-fluorone and 1.27 g (10.0 mmol) of iodine in 50 ml of absolute ethanol at 0°C. This mixture was stirred over two hours then 50 ml of cool water was added. The precipitate which formed was filtered and washed with a small amount of ethanol. 2,7-di-t-butyl-yield. HNMR (DMSO): 8.21(s,1H), 7.55(s,2H), 1.38(s,18H).

Synthesis Example 6

2,7-Di-t-butyl-4,5-disulfurnyl-3-hydroxy-6-fluorone

Fuming sulfuric acid (3.0 ml of 15-18%) was added dropwise to 2,7-di-t-butyl-3-hydroxy-6-fluorone (1.6 g, 4.9 mmol.). The mixture was stirred until the compound dissolved it was slowly warmed to 40-50∘C for half hour. Ice (10-15 g) was carefully added to the resulting mixture at 0∘C with stirring. The precipitate which formed was filtered and 2,7-di-t-butyl-4,5-disulfurnyl-3-hydroxy-6-fluorone (1.72 g) was obtained in 72% yield. HNMR (DMSO): 9.7(s,1H), 8.17(s,2H), 1.40(s,18H).

Synthesis Example 7

3,6-Dihydroxy-4,5-diiodoxanthane.

To a suspension of 3,6-dihydroxy-4,5-diiodoxanthone (9.6 g, 20.0 mmol) in 100 ml of THF was added 40 ml of boran-tetrahydrofuran complex (1.0 M in THF) at room temperature under nitrogen. After stirring for 5 hours at r.t., the reaction mixture was quenched by adding 20.0 ml of 0.5 N HCl. After stirring for 30 minutes the solvent was removed. The residue was filtered and the precipitate was dissolved in the 0.1 N NaOH solution. This basic solution was filtered and acidified with 0.5 N HCl. The yellow solid product which formed was filtered. 3,6-dihydroxy-4,5-diiodoxanthane (9.2 g, 96.6%) was obtained. HNMR (DMSO): 10.38-(s,2H), 2.05 (d,2H, J = 8.3 Hz), 6.67(d,2H, J = 7.8 Hz), 3.88(s,2H).

Synthesis Example 8

4,5-Diiodo-3-hydroxy-6-fluorone.

To a solution of 3,6-dihydroxy-4,5-diiodoxanthane (233 mg 0.5 mmol) in 10 ml of ethanol at 25∘C was added 2,3-dichloro-5,6-dicyano-1,4-benzoquinone (DDQ) (113 mg, 0.5 mmol). The reaction mixture was stirred for 4 hours at room temperature. The yellow precipitate was filtered and washed with ethanol. 4,5-Diiodo-3-hydroxy-6-fluorone (180 mg) was obtained in 77.6% yield. HNMR (DMSO): 8.22(s,1H), 7.68(d,2H, J = 8.8 Hz), 6.84(d,2H, J = 8.3 Hz).

Synthesis Example 10

General procedure for preparation of 9-cyano substituted xanthene derivatives:

Potassium cyanide (2.0 mmol) was added to a solution of a 9-hydrogen substituted xanthene (1.0 mmol) in 5.0 ml of DMF. The reaction was monitored by the visible absorption spectrum until the starting material was completely consumed. The reaction solution was treated with 4:1 hexane:dichloromethane after which the precipitate which formed was filtered, dried and treated with 5% HCl and washed with water. 9-Cyano substituted fluorone derivatives were obtained in 80-95% yield. Analytically pure compounds were

isolated by TLC separation.

| Compound | HNMR (DMSO) |
|---|---|
| 9-Cyano-3-hydroxy-6-fluorone | 7.28(d,2H, J=9.24 Hz), 6.28 (dd,2H, J=9.24, 1.92 Hz), 5.94 (d,2H, J=1.98 Hz) |
| 9-Cyano-2,4,5,7-tetra-t-butyl-3-hydroxy-6-fluorone | 7.79(s,2H) |
| 9-Cyano-2,4,5,7-tetraiodo-3-hydroxy-6-fluorone | 8.01(s,2H) |
| 9-Cyano-4,5-diiodo-3-hydroxy-6-fluorone | 7.38(d,2H, J=9.2 Hz), 6.46 (d,2H, J=9.2 Hz) |
| 9-Cyano-2,7-Di-t-butyl-3-hydroxy-6-fluorone | 7.41(s,2H), 1.37(s,18H) |
| 9-Cyano-2,7-di-t-butyl-4,5-diiodo-3-hydroxy-6-fluorone | 7.28(s,2H), 1.34(s,18H) |

Synthesis Example 11

2,4,5,7-Tetraiodo-3-hydroxy-6-fluorone (TIHF)

Iodic acid (16.4 g, 93 mmol) dissolved in a minimum amount of water (app. 1 ml per gram) was added dropwise over 20 min. to a solution of 10 g (46.7 mmol) of 3,6-dihydroxyxanthane and 29.8 g (118 mmol) of iodine in 1 L of absolute ethanol. This mixture was stirred over two hours during which the dark brown solution slowly turned a red orange as the solid initiator precipitated. The mixture was then warmed for an hour at 60∘C. After cooling, the mixture was filtered, washed with water and ethanol and the crude solid tritiated with absolute ethanol and refiltered to give, after drying under vacuum, 29 g (87%) of a red solid (λ=536 nm in ethanol) $H^1$ NMR (DMSO) 8.31 (s, 2H), 8.07 (S, 1H).

Synthesis Example 12

2,4,4,7-Tetrabromo-3-hydroxy-6-fluorone (TBHF)

To a solution of 3,6-dihydroxyxanthane (4.28 g 20 mmol) in 150 ml of ethanol was added sodium hydroxide solution (4.8 g, 120 mmol in 10 ml of $H_2O$). The solution was cooled to 0∘C then $Br_2$ (22.4 g, 140 mmol) was slowly added. After addition, the reaction mixture was stirred for 8 hrs. at room temperature. Precipitate was filtered off and washed with ethanol. 2,4,5,7-tetrabromo-3-hydroxy-6-fluorone (9.6 g 90.8%) was obtained. NMR (DMSO) 8.309 (s, 1H), 8.244 (s, 2H, λmax=526 nm (methanol).

In the table below, the properties of the foregoing initiators are shown:

| Compound | max[a] | f[b] | f | pKa | Eox | Ered | phos |
|---|---|---|---|---|---|---|---|
| HF | 500,504,490 | 506,513 | .95[c] | 5.97 | 1.34 | -.99 | --- |
| TBHF | 526,530,516 | 539 | .52 | 3.28 | 1.099 | -.95 | --- |
| TIHF | 532,536,526 | 544,549 | .13 | 4.08 | 1.04 | -.95 | 676 |

Entries in the table are in the solvents according to the following order.

a. MeOH, EtOH, 10% MeOH/90% water

b. MeOH, EtOH

c. $10^{-7}$ M

Synthesis Example 13

2,4,5,7-Tetraiodo-9-cyano-3-hydroxy-6-fluorone

Potassium cyanide (1.175 g, 18.0 mmol) was added to a solution of TIHF (10.74 g 15.0 mmol) in 100 ml of DMF. The reaction was monitored by its visible spectrum until all of the TIHF was consumed. The solvent was removed with a vacuum pump at room temperature and the residue was treated with 1:1 hexane/dichloromethane. The precipitate was filtered, dried and treated with 18% HCl then washed with water. 2,4,5,7-tetralodo-9-cyano-3-hydroxy-6-fluorone (10.60 gh) was obtained (975). HNMR (DMSO):8.006-(2H,s) max (EIOH = 638 nm).

Synthesis Example 14

6-Hydroxy-3-methoxyxanthone

7.0 g (30.7 mmol) of 3,6-dihydroxyxanthone is dissolved in aq. NAOH (2.5 g (62.5 mmol) in 200 mls $H_2O$). 3.2 mls (33.6 mmol) of dimethysulfate is added dropwise with stirring, and solid forms immediately upon completion. Stirred further 1 hour and heated to reflux for 15 minutes. Additional base is added to insure complete solubility of phenols. Filtration yields 1. Solution is acidified with HCl, and filtration yields a mixture of staring material and 4. Solid is heated to reflux in about 200 mls ethanol for 15 minutes to dissolving starting material, and solid is filtered out, washing with ethanol and water. This may have to be repeated several times to obtain pure 4 in about 50% yield; mp = 303-306-C.NMR (200 $MH_z$ in d-DMSO) s 3.89 (3H,s), 6.82 (1H,d,J = 2.24 $H_z$), 6.87 (1H,m), 6.99 (1H,dd,J = 8.78 $H_z$, 2.20 $H_z$), 7.09 (1H,d,J = 2.26$H_z$), 7.99 (1H,d,J = 8.52$H_z$), 8.03 (1H,d,J = 8$H_z$).

Synthesis Example 15

6-Hydroxy-3-methoxyxanthane

To a solution of 4.0 g (16.5 mmol) of 4 in 150 mls of anhydrous THF under nitrogen is added with stirring 41 mls (41 mmol) of 1 M $BH_3$ in THF. Reaction mixture is stirred overnight at room temperature. Solvent is stripped and flask washed out with aq. NaOH, dissolving product completely. After filtration to remove impurities, the solution is acidified, filtered and washed with water to yield 3.73 g (99%) of yellow 5. NMR (200 $MH_z$ in d-DMSO) s 3.72 (3H,s), 3.82 (2H,s), 6.46 (2H,M), 6.63 (2H,m), 7.00 (1H,d,J = 8.12$H_z$), 7.10 (1H,d,J = 9.46 $H_z$). MS: 227,212,197,184,128.

Synthesis Example 16

5,7-Diiodo-3-methoxy-6-fluorone

To a solution of .228 g (1 mmol) of 5 and .508 g (4 mmol) of iodine in 20 mls of ethanol, .211 g (1.2 mmol) of iodic acid in minimum water is added dropwise with stirring 1 hour at room temperature then warmed to 5- - C for 15 minutes. Filtration and washing with ethanol and water yields .44 g (92%) of orange 6. NMR (200 $MH_z$ in d-DMSO) s 3.96 (3H,s), 7.10 (1H,dd,J = 8.72 $H_z$, 2.38 $H_z$), 7.19 (1H,d,J-2.38), 7.86 (1H,d,J = 8.74$H_z$), 8.35 (1H,s), 8.46 (1H,s). MS: 478,450,351,323,196,181,125.

As the foregoing examples and Reaction Scheme 1 illustrate, the 2- and 7- positions can be functionalized by an electrophilic substitution reaction. Subsequently, the 9-position is functionalized by a 1,6-conjugated addition reaction followed by autoxidation. See for example compounds 8 and 9 in Reaction Scheme 1. Introduction of functional groups at the 4 and 5 positions may also be accomplished by electrophilic substitution. From 2,7-di-t-butyl-3-hydroxy-6-fluorone, compound 5 in Reaction Scheme 1, 2,7-di-t-butyl-3-hydroxy-4,5-diiodo-6-fluorone can be prepared by reaction with iodine and $HIO_3$. 2,7-Di-t-butyl-3-hydroxy-4,5-disulfurnyl-6-fluorone can be prepared from compound 5 by reaction with 15-18% fuming sulfuric acid. Each of these derivatives can be reacted with KCN in DMF using the procedure of Synthesis Example 10 to yield the corresponding 9-cyano derivative.

The 4- and 5-positions can also be functionalized as shown in Reaction Scheme 3.

Reaction Scheme 3

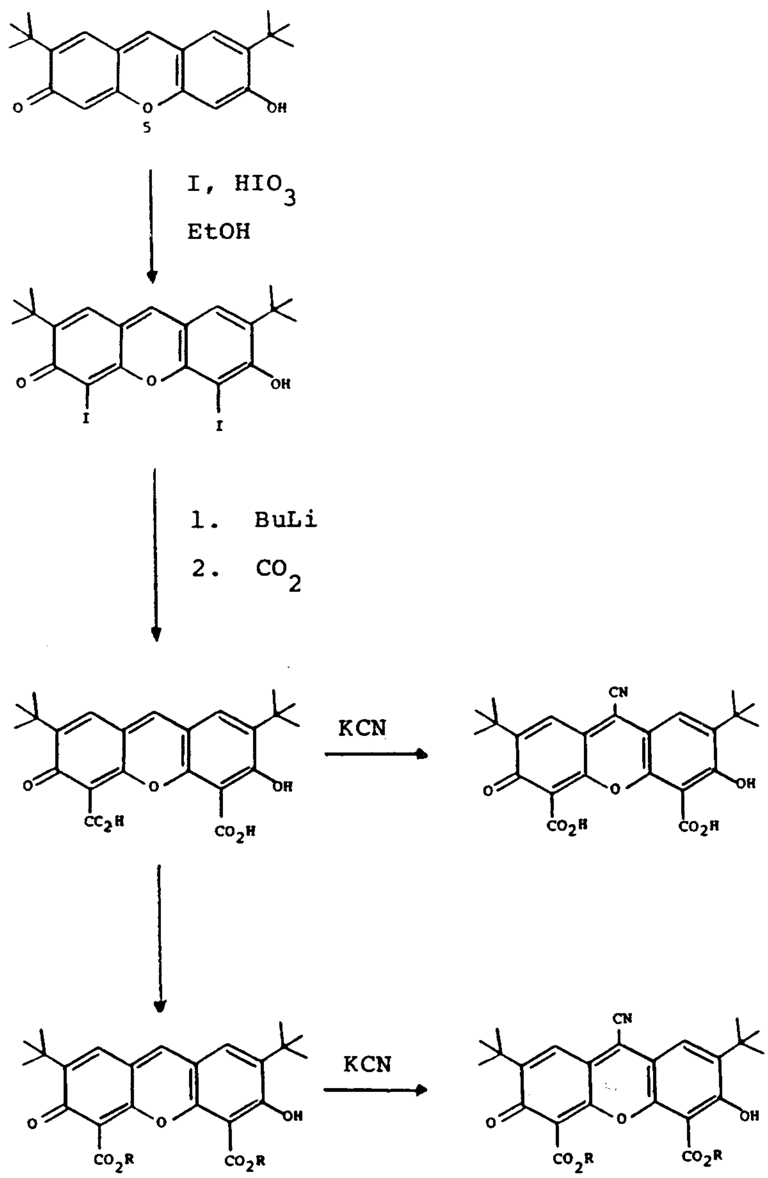


Many other 4,5 derivatives can be prepared from 3-hydroxy-4,5-diiodo-6-fluorone (DIHF) which can be prepared as shown in Reaction Scheme 4 below:

Reaction Scheme 4

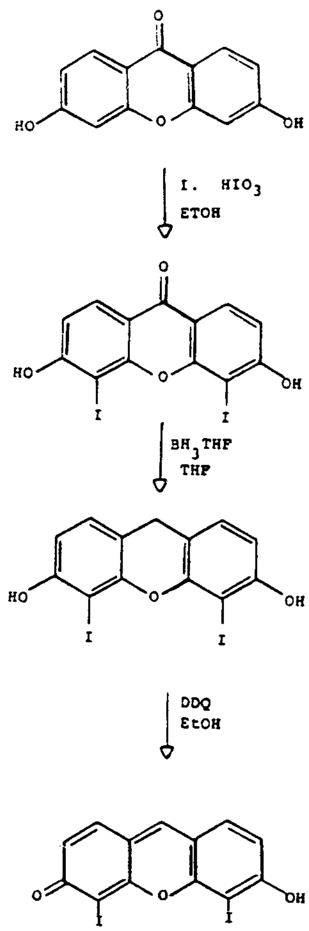


Compounds of the general formula (IX) may be obtained by aminomethylation of the corresponding fluorone followed by functionalization of the 9-position or by functionalization of the following compound using reactions which are analogous to those shown in Reaction Scheme 1 for the fluorones.

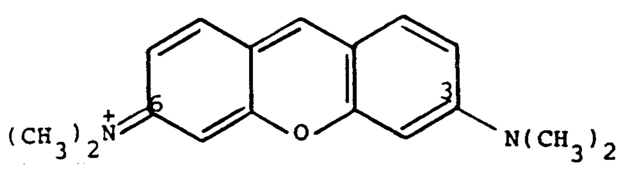

Compounds of the formula XI can be prepared by the following Reaction Scheme 5:

Reaction Scheme 5

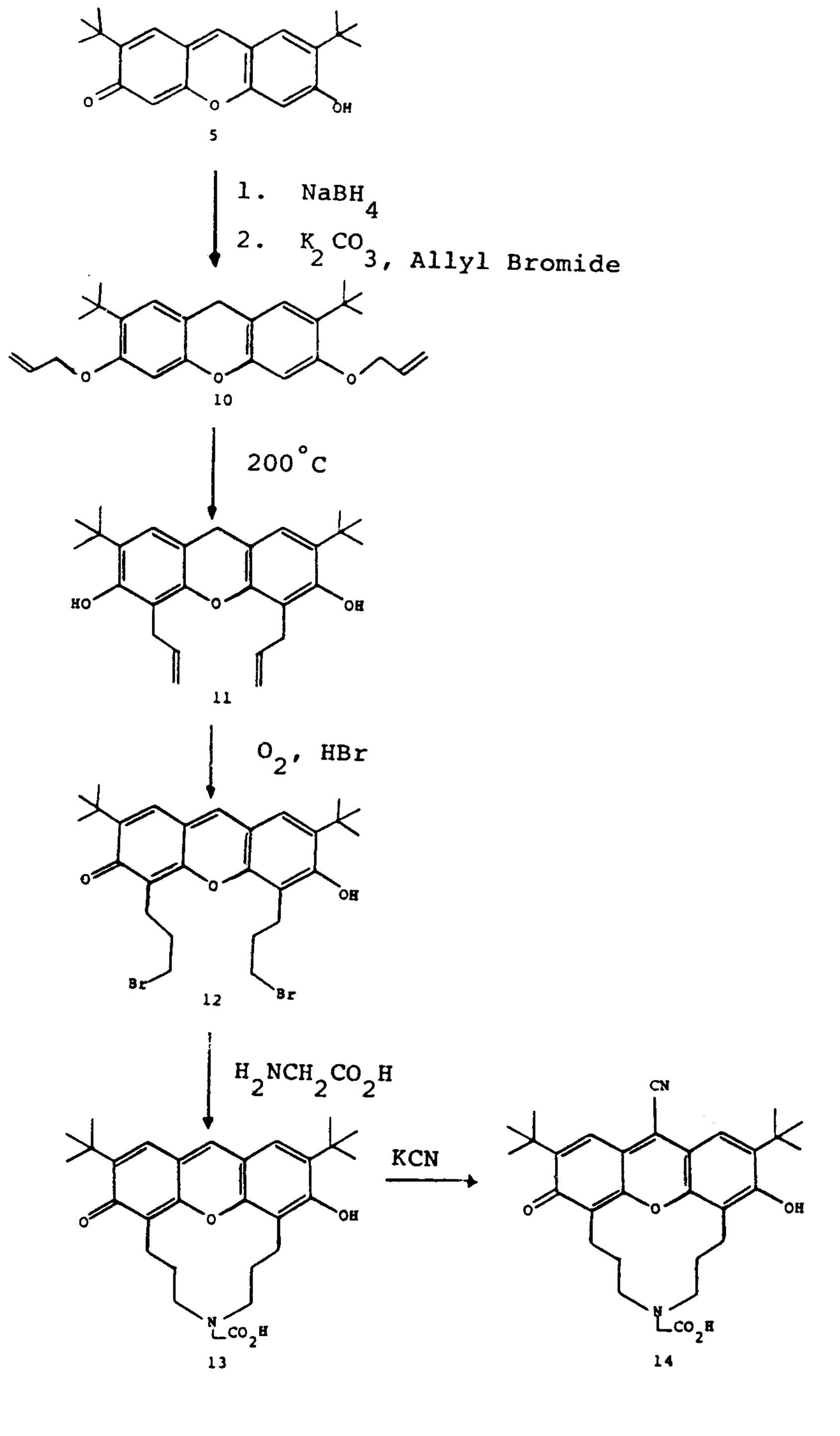


A proposed synthesis for 9-trifluoromethyl derivatives is provided in Reaction Scheme 6:

Reaction Scheme 6

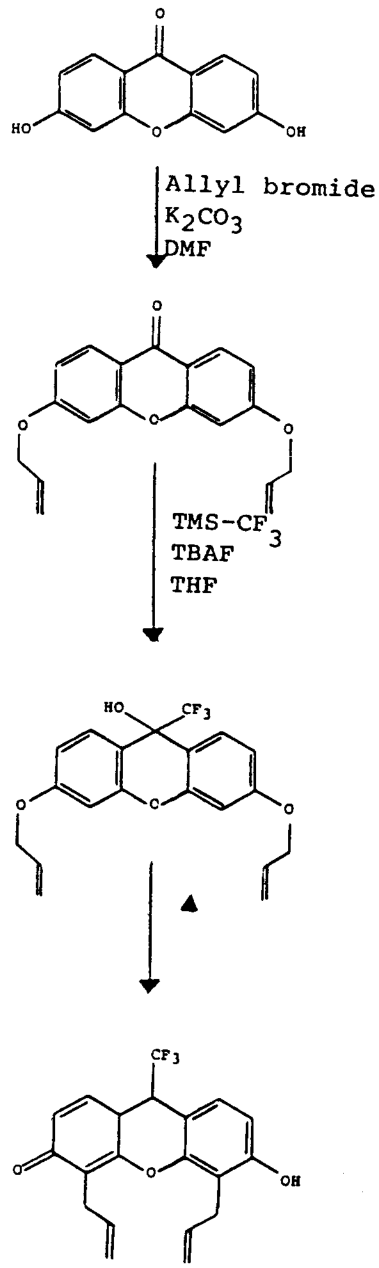


When $R^7$ is H in formulas III and IV, the compounds are herein referred to as dehydroxfluorones. A proposed synthetic route for dehydroxyfluorones is shown in Reaction Scheme 6.

Examples of such compounds are shown below:

COMPOUND A

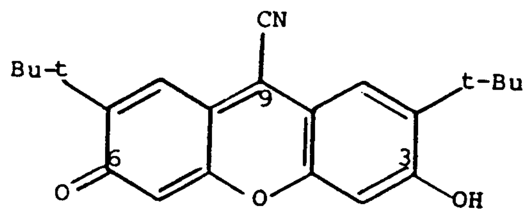

COMPOUND B

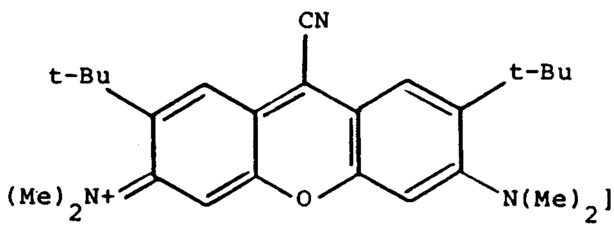

COMPOUND C

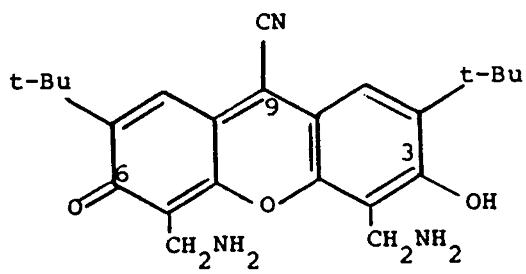

COMPOUND D

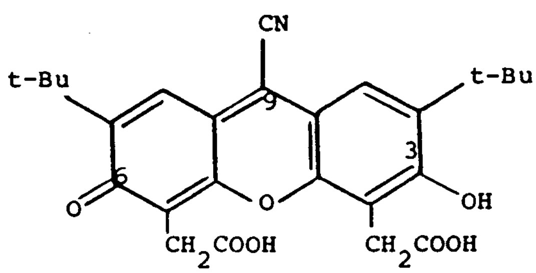

COMPOUND E

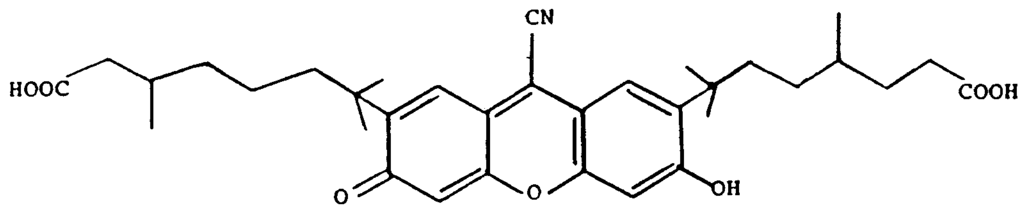

COMPOUND F

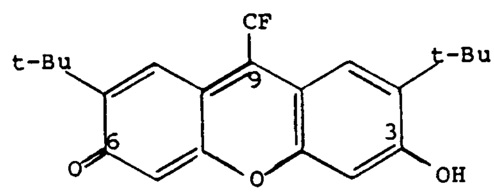

COMPOUND G

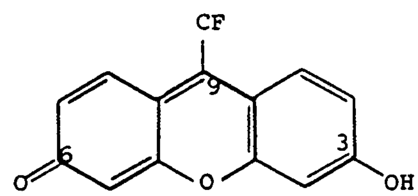

COMPOUND H

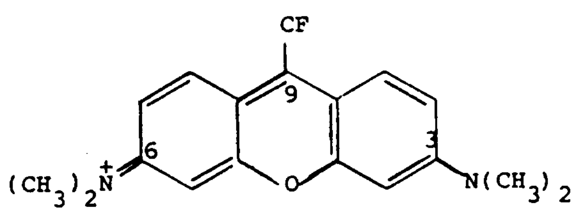

COMPOUND I

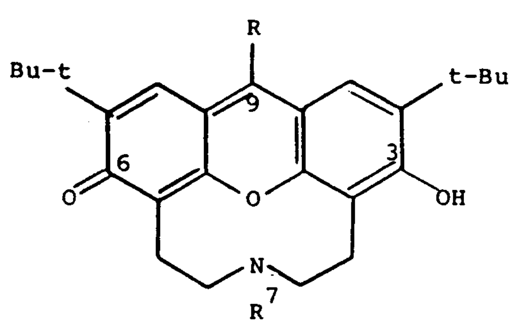

R=H.CN.$CF_3$.
$R^7$=$(CH_2)_n$COOH,
$(CH_2)_nNH_2$
n= 1-6

## Example 1

The following compositions were prepared:

| Composition 1 | |
|---|---|
| TMPTA | 90 g |
| NVP | 10 g |
| TIHF | 3.6 mg |
| NPG | 75 mg |

| Composition 2 | |
|---|---|
| DPHPA | 87 g |
| NVP | 13 g |
| NPG | 3.6 mg |
| TIHF | 75 mg |

| Composition 3 | |
|---|---|
| DPHPA | 30 G |
| NVP | 10 g |
| TIHF | 3.6 mg |
| NPG | 75 mg |
| TMPTA | 20 g |
| Ebecryl 370 (bisphenol A diacrylate) | 4 oz |

| Composition 4 | |
|---|---|
| NVP | 10 g |
| TIHF | 3.6 mg |
| NPG | 75 mg |
| Tetraethylene glycol diacrylate | 90 g |

Results

Each composition was placed in a test tube and exposed to a broad band light source. The compositions all cured rapidly.

Examples of our compounds are useful in many of the same applications as conventional photoinitiators and fluorescent dyes. In particular, they are useful as a substitute for fluorescein dyes and other fluorescent agents in immunoassays and still more particularly in polarization immunoassays of the type described in U.S. Patent 4,585,862. The compounds are advantageous because they absorb at much longer wavelengths than conventional fluorescers and this enables the use of inexpensive lasers such as He/Ne lasers to excite the compounds. Furthermore, because of their long absorption wavelengths, it may be possible to perform immunoassays on whole blood using these compounds. Presently, it is generally necessary to remove the blood cells from the blood in order to prevent them from absorbing the excitation energy.

Examples of our compounds are also useful as fluorescent oil additives for use in engine leak detection. Presently, the fluorescent dyes used in this process require the use of high powered lamps to excite the dye and in order to produce a sufficient level of fluorescence to detect oil leakage due to the competing absorption of the oil and the oil additives. Because of our compounds absorb at longer wavelengths, less competing absorption is encountered and lower power light sources can be used to detect oil leakage. The other components of the oil do not compete as much for the light at longer wavelengths. A particularly preferred fluorescer for use in leak detection is 2,7-di-t-butyl-3-hydroxy-6-fluorone, Compound No. 5 in Reaction Scheme 1 above.

A particularly useful class of fluorescers are compounds of the formula IV in which $R^1$ and $R^2$ are substituted and $R^3$ and $R^4$ are hydrogen. For use in immunoassay, ionic moieties can be added to the

compounds to enhance their water solubility. However, in the fluorescent compounds $R^1$-$R^4$ are usually not a heavy atom such as bromine or iodine. Fluorescence has not been observed with the latter compounds. However, they are effective photoinitiators. As a general rule in order to achieve fluorescence, the compound must be ionized. In nonpolar media such as oils, it may be necessary to include a functional group in $R^1$-$R^4$ which interacts with W/W' to produce intramolecular ionization.

The fluorone initiators are preferably used in combination with a coinitiator. Useful coinitiators can be selected from among those known in the art and, more particularly, from known electron donating coinitiators. N-phenylglycine (NPG) is a well known and preferred coinitiator. Examples of other useful electron donating coinitiators are discussed in Eaton, D.F., "Dye Sensitized Photopolymerization", Advances in Photochemistry, Vol. 13, pp. 427-486. Also useful are N,N-dialkylanilines and other tertiary amines such as diethanolamine, triethanolamine, and arylsulfinates.

Dialkylanilines appear to function as autoxidizers and are preferably used in combination with an electron donor such as NPG. Representative examples of useful N,N-dialkylanilines are 4-cyano-N,N-dimethylaniline, 4-acetyl-N,N-dimethylaniline, 4-bromo-N,N-dimethylaniline, 4-methyl-N,N-dimethylaniline, 4-ethoxy-N,N-dimethylaniline, N,N-dimethylthioanicidine, 4-amino-N,N-dimethylaniline, 3-hydroxy-N, N-dimethylaniline, N,N,N'N'-tetramethyl-1,4-dianiline, 4-acetamido-N,N-dimethylaniline, 2,6-diethyl-N,N-dimethylaniline, N,N,2,4,6-pentamethylaniline (PMA) and p-t-butyl-N,N-dimethylaniline.

Certain other tertiary amines are also useful coinitiators including triethylamine, triethanolamine, etc.

Another class of useful coinitiators is alkyl borate salts such as ammonium and pyridinium salts of borate anions of the formula $BR^{18}R^{19}R^{20}R^{21}$ where $R^{18}$-$R^{21}$ are independently selected from the group consisting of alkyl, aryl, alkaryl, allyl, aralkyl, alkenyl, alkynyl, alicyclic and saturated or unsaturated heterocyclic groups.

Representative examples of alkyl groups represented by $R^{18}$-$R^{21}$ are methyl, ethyl, propyl, butyl, pentyl, hexyl, octyl, stearyl, etc. The alkyl groups may be substituted, for example, by one or more halogen, cyano, acyloxy, acyl, alkoxy or hydroxy groups. Representative examples of aryl groups represented by $R^{18}$-$R^{21}$ include phenyl, naphthyl and substituted aryl groups such as anisyl and alkaryl such as methylphenyl, dimethylphenyl, etc. Representative examples of aralkyl groups represented by $R^{18}$-$R^{21}$ include benzyl. Representative alicyclic groups include cyclobutyl, cyclopentyl, and cyclohexyl groups. Examples of an alkynyl group are propynyl and ehtynyl, and examples of alkenyl groups include a vinyl group.

Preferably, at least one but not more than three of $R^{18}$-$R^{21}$ is an alkyl group. Each of $R^{18}$-$R^{21}$ can contain up to 20 carbon atoms, and they typically contain 1 to 7 carbon atoms. More preferably $R^{18}$-$R^{21}$ are a combination of alkyl group(s) and aryl group(s) or aralkyl group(s) and still more preferably a combination of three aryl groups and one alkyl group, i.e., an alkyltriphenylborate. Useful salts are disclosed in U.S. Patent 4,950,581.

Presently, a combination of N-phenylglycine (NPG) and diisopropyldimethylaniline (DIDMA) is used as coinitiator when high speed is required, for example, in a black radiation curable ink.

The most typical example of a free radical addition polymerizable or crosslinkable compound with which the initiator and coinitiator are useful is an ethylenically unsaturated compound and, more specifically, a polyethylenically unsaturated compound. These compounds include both monomers having one or more ethylenically unsaturated groups, such as vinyl or allyl groups, and polymers having terminal or pendant ethylenic unsaturation. Generally, any unsaturated compound which is convertible to a solid by free radical polymerization is useful. Such compounds are well known in the art and include acrylic and methacrylic esters of polyhydric alcohols such as trimethylolpropane, pentaerythritol, and the like; and acrylate or methacrylate terminated epoxy resins, acrylate or methacrylate terminated polyesters, etc. Representative examples include ethylene glycol diacrylate, ethylene glycol dimethacrylate, trimethylolpropane triacrylate (TMPTA), pentaerythritol tetramethacrylate, dipentaerythritol hydroxypentacrylate (DPHPA), hexanediol-1,6-dimethacrylate, hexanediol diacrylate, diethyleneglycol dimethacrylate, diglycidyl ether diacrylate (MW = 390), trimethylol propane ethoxylate triacrylate, neopentyl glycol propoxylate diacrylate (MW = 428). Other vinyl compounds such a N-vinyl pyrrolidone (NVP) are also useful. The monomers may be selected and blended to provide the optimum photographic speed and/or the physical characteristics desired in the product. Monomer selection and blending may also be used to insure adequate solubility of the photoinitiators. A particularly preferred monomer composition is 87% DPHPA and 13% NVP. Another preferred composition is TMPTA, DPHPA and NVP>

It may be desirable to incorporate a sensitizer in the composition to control the sensitivity of the composition and/or to extend its sensitivity. Useful sensitizers include those known in the art such as anthracene, and its derivatives naphthalene, acetophenone, benzophenone, 2-acetonaphthone, etc.

Solvents may be necessary to dissolve the photoinitiator if the photoinitiator is not sufficiently soluble in the monomer. Solvents may also be used to shift the absorption spectrum to tune the sensitivity of the

composition. Some examples of useful solvents are N-vinylpyrrolidone and nitrobenzene. Other useful solvents can be identified readily.

The nature of the monomer, the amount of the fluorone and coinitiator in photohardenable compositions will vary with the particular use of the compositions, the emission characteristics of the exposure sources, the development procedures, the physical properties desired in the polymerized product and other factors. With this understanding, compositions will generally fall within the following compositional ranges in parts by weight (based on 100 parts total).

| | |
|---|---|
| Polymerizable compound | 50 to 99.7 |
| Fluorone | .02 to .05 |
| Electron Donor | 0.2 to 1 |
| Autoxidizer | 0 to 3 |
| Sensitizer (optional) | 0.3 to 1 |

Compositions may typically have the following formulation:

| | |
|---|---|
| Polymerizable compound | 10 g |
| Fluorone | 2 mg to 10 mg |
| Electron Donor | 20 mg to 100 mg |
| Autoxidizer | 0 to 3 g |
| Sensitizer (optional) | 20 mg to 100 mg |

The photohardenable compositions can be coated upon a support in a conventional manner and used in making a photoresist or in photolithography to form an oleophilic polymer image. Development of the photohardenable compositions is conducted in an otherwise known or conventional manner, e.g., a solvent for the unpolymerized monomer may be used to remove the photohardenable composition in the unexposed areas.

The compositions can also be encapsulated as described in U.S. Pat. Nos. 4,399,209 and 4,440,846 and used to control the release of an image-forming agent. The latter processes typically involve imagewise exposing the photosensitive layer to actinic radiation and subjecting the layer of microcapsules to a uniform rupturing force such as pressure.

The photohardenable composition is also advantageous for use in the three dimensional modeling process taught in U.S. Pat. 4,575,330 to Hull. Due to the thicker depth of cure that is possible, models may be prepared in larger cross-sectional increments. This should reduce the total time required for the model building process. Another advantage which the claimed compounds bring to three dimensional modeling is higher green strength. Depending upon the extinction coefficient, the compositions and photosensitive materials can be exposed to any source which emits in this range and particularly an He/Cd laser, or mercury arc lamps.

We also contemplate use of our fluorones in dosimeters and more particularly in dosimeters useful in detecting UV, visible and infrared laser pulses. Because fluorones undergo a color change when exposed to radiation, they may be used to record and provide a quantitative indication of exposure to actinic radiation. For this purpose, they may be dispersed in a compatible binder such as polyvinyl alcohol or polymethyl methacrylate and coated upon a support. When the color change alone is not suitable for use in dosimetry, the photohardenable compositions described above may be useful. Exposure of the compositions produces a change which is indicative of the type or amount of exposure.

In addition to being useful in photosensitive materials, the compositions are also useful in a wide variety of other applications including photocurable inks and coatings, photoadhesives, printing plates, printed circuit fabrication, and other applications for photohardenable compositions.

While we have referred to free radical polymerizable compositions, it is anticipated that the fluorones described herein are also useful in ionically polymerizable compositions and more particularly cationically polymerizable compositions. In this arrangement, the fluorones are used with onium salts such as iodonium, phosphonium, sulfonium and pyrylium slats. Cationically polymerizable compositions based upon onium slats are known in the art as shown in U.S. Patents 4,264,703 and 4,307,177 to Crivello, and European Application 0 408 227 A1.

**Claims**

1. A compound of the formula:

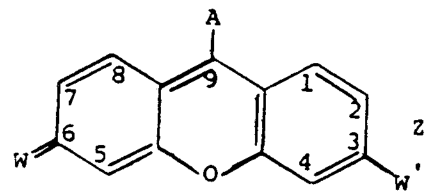

where A is hydrogen, alkyl, alkenyl, alkynyl, dichlorotriazinylamino, or an electron withdrawing group-(EWG) which is more strongly electron withdrawing than a phenyl or a substituted phenyl group, W and W' are respectively selected from O and $O^-$, or $NR_2^+$ and $NR_2$, R is hydrogen, lower alkyl, aralkyl, or aryl, and Z is a hydrogen atom or a counter ion, and the compound may be substituted or unsubstituted at the 2, 4, 5 and 7 positions, provided that when A is hydrogen or methyl, the compound is substituted at at least one of the 2, 4, 5, and 7 positions.

2. A compound according to Claim 1, further characterised in that A is selected from hydrogen, alkenyl, alkynyl, dichlorotriazinylamino, COOR, $COR^5$, $CONR_2$, C(O)OCOR, CN, $NO_2$, NCS, NCO, $SO_2R^5$, $SO_3R$, $SO_2NR_2$, $CX_3$ where R is hydrogen, alkyl, aryl, or aralkyl, X is a halogen atom; and $R^5$ is hydrogen, alkyl, aryl, aralkyl, N-imido or $NR_2$.

3. A compound according to Claim 2, further characterised in that said compound is represented by the formula (V):

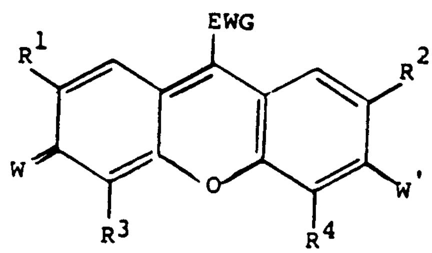

(V)

where EWG is an electron withdrawing group consisting of A as defined in Claim 2; $R^1$-$R^4$ may be the same or different and are selected from hydrogen, halogen, alkyl, alkenyl, aryl, aralkyl, alkaryl, $(CH_2)_nCOOR^5$, $(CH_2)_nCONR_2$, $(CH^2)_nX$, $SO_3R$, $SO_2R^5$, $SO_2NR_2$, $NO_2$, $(CH_2)_nNR_2$, NCO, NCS, $(CH_2)_nNCO$, $(CH_2)_nNCS$, CN, $(CH_2)_nCN$, where n is 1 to 6, and $R^3$ is $R^4$ may combine to form a 10 to 12 membered ring; and $R^5$ is hydrogen, alkyl, aryl, aralkyl, N-imido or $NR_2$.

4. A compound according to Claim 3, further characterised in that $R^1$ and $R^2$ are alkyl groups.

5. A compound according to Claim 3, further characterised in that $R^1$ and $R^2$ are alkyl groups, W is $NR_2^+$ and W' is $NR_2$.

6. A compound according to Claim 3, further characterised in that $R^1$ and $R^2$ are alkyl groups, W is O and W' is O-.

7. A compound according to any of Claims 1, 5 or 6, further characterised in that EWG is CN.

8. A compound according to Claim 3, further characterised in that EWG is $CF_3$.

9. A compound according to Claim 3, further characterised in that $R^3$ and/or $R^4$ includes a functional

group suitable for coupling said compound to a ligand-analog or an antibody.

**10.** A compound according to Claim 9, further characterised in that said functional group is $COOR^5$ or $NR_2$.

**11.** A compound according to Claim 10, further characterised in that said functional group is oxalyl, sulfonyl, N-imido or carboamidosulfonyl.

**12.** A compound according to Claim 3, further characterised in that at least one of $R^1$-$R^4$ includes a functional group which products intramolecular ionization of said compound.

**13.** A compound according to Claim 3, further characterised in that A is hydrogen and one of $R^1$-$R^4$ is CN, NCO, NCS, $(CH_2)_nCN$, $(CH_2)_nNCO$ and $(CH_2)_nNCS$ where n is 1 to 6.

**14.** A compound according to any preceding claim, further characterised in that said compound fluoresces upon the absorption of actinic radiation.

**15.** A compound according to any of Claims 1 to 13, wherein said compound is capable of generating free radicals upon absorption of actinic radiation alone or in combination with a coinitiator.

**16.** A compound of the formula (X):

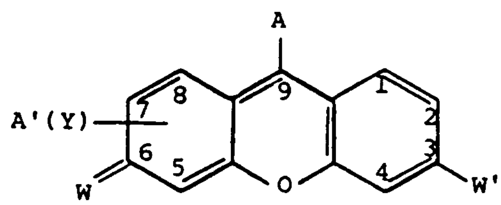

(X)

where A is selected from hydrogen,
alkenyl, alkynyl, dichlorotriazinylamino, COOR, $COR^5$, $CONR_2$, C(O)OCOR, CN, $NO_2$, NCS, NCO, $SO_2R^5$, $SO_3R$, $SO_2NR_2$, $CX_3$ where R is hydrogen, alkyl, aryl, or aralkyl and X may be the same or different and is a halogen atom; W and W' are selected from either O and O-, or $NR_2$ + and $NR_2$; $R^5$ is hydrogen, alkyl, aryl, aralkyl, N-imido or $NR_2$; and A' represents a ligand-analog or an antibody and Y is a linking group and the A'(Y) moiety may be at one of the 2, 4, 5, 7 or 9 positions and the balance of those positions may be substituted or unsubstituted.

**17.** A composition for detecting oil leakage comprising an oil vehicle and a fluorescent dye, characterised in that said dye is a compound of the formula:

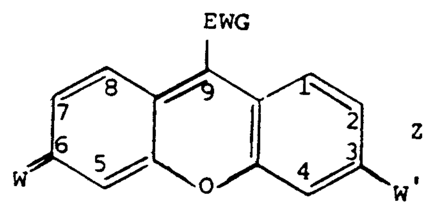

where EWG is an electron withdrawing group which is more strongly electron withdrawing than a phenyl or a halogen or carboxyl substituted phenyl group, W and W' are O and $O^-$ or $NR_2^+$ and $NR_2$, R is hydrogen, alkyl, aralkyl, or aryl, Z is a hydrogen atom or a counter ion, and the compound is substituted with an oleophilic moiety at at least one of the 2, 4, 5 and 7 positions.

**18.** A compound of the formula (III) or (IV)

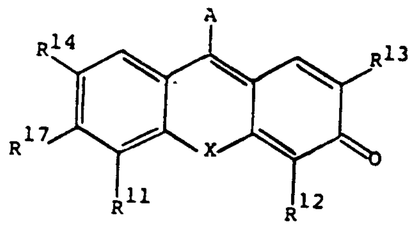

(III)

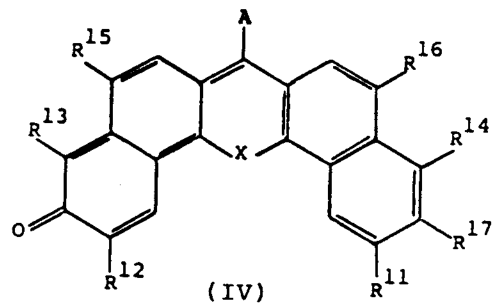

(IV)

where X is oxygen, sulfur, selenium, tellurium, or C = 0, A is hydrogen, alkenyl, aryl, or an electron withdrawing group selected from quaternary amino,
nitro, nitroso, cyano, $-SO_3R'$, $-SO_2R'$, halogen,
trihalomethyl, -CHO, carbamoyl, sulfamoyl, sulfinyl and -COOR'

where R' is hydrogen, alkyl, aryl, or aralkyl; and $R^{11}$-$R^{16}$ are the same or different and represent a hydrogen or a halogen atom; and $R^{17}$ is a hydrogen atom or an -OR' group.

**19.** A compound according to Claim 18, further characterised in that said fluorone is represented by the formula VI

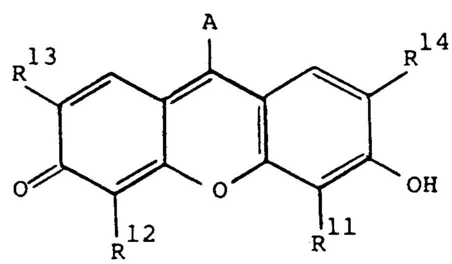

**20.** A compound according to Claim 19, further characterised in that A is hydrogen and $R^{11}$-$R^{14}$ are bromine.

**21.** A compound according to Claim 19, further characterised in that it is useful as a photoinitiator.